# EUROPÄISCHE PATENTSCHRIFT

(11) **EP 2 957 273 B1**
(45) Veröffentlichungstag und Bekanntmachung des Hinweises auf die Patenterteilung: **12.07.2017**
(21) Anmeldenummer: 15168233.3
(22) Anmeldetag: 19.05.2015
(51) Int. Cl.: A61J 1/16

(54) **FLASCHENHALTER FÜR EINE INJEKTIONS- ODER INFUSIONSVORRICHTUNG**
BOTTLE HOLDER FOR AN INJECTION OR INFUSION DEVICE
SUPPORT DE BOUTEILLES POUR UN DISPOSITIF D'INFUSION OU D'INJECTION

(30) Priorität: 16.06.2014 DE 102014108452
(43) Veröffentlichungstag der Anmeldung: 23.12.2015
(73) Patentinhaber: Ulrich GmbH & Co. KG, 89081 Ulm (DE)
(72) Erfinder: Bückle, Norbert, 89182 Bernstadt (DE)
(74) Vertreter: Charrier Rapp & Liebau

(56) Entgegenhaltungen:
- ES-A1- 2 397 476
- US-A1- 2011 266 409

## Beschreibung

Die Erfindung betrifft einen Flaschenhalter für eine Injektions- oder Infusionsvorrichtung nach dem Oberbegriff des Anspruchs 1.

Aus dem Stand der Technik sind Injektionsvorrichtungen bekannt, welche im Bereich der Medizintechnik zur Injektion von Flüssigkeiten in den Körper eines Patienten verwendet werden. Solche Injektionsvorrichtungen können beispielsweise zur Verabreichung von Kontrastmitteln bei der Durchführung von bildgebenden Verfahren wie Computertomographie, Ultraschalluntersuchungen und Magnetresonanztomographie (MRT) verwendet werden. Die zu injizierenden Flüssigkeiten, wie z.B. verschiedene Kontrastmittel und NaCl-Spüllösungen, sind dabei in Vorratsflaschen abgefüllt. Die Flaschen mit den zu injizierenden Flüssigkeiten werden bspw. am oberen Ende eines in der Regel rollengelagerten Ständers aufgehängt oder in einem Flaschenhalter mit einer Flaschenaufnahme gelagert und über einen Zufuhrschlauch mit einer Injektionsvorrichtung verbunden.

Eine derartige Vorrichtung ist aus der US 6,070,761 A bekannt. Diese weist mehrere Flaschenhalter für Vorratsflaschen auf, wobei sich jeder Flaschenhalter aus zwei äußeren Armen und zwei inneren Armen zusammensetzt. Die äußeren Arme sind starr an einem Panel angebracht und weisen eine bogenförmige Flaschenaufnahme zur Aufnahme eines Kopfs bzw. eines Flaschenhalses einer Vorratsflasche auf. In den äußeren Armen sind die inneren Arme mit einer ebenfalls bogenförmigen Flaschenaufnahme zur Aufnahme des Flaschenhalses der Vorratsflasche verschwenkbar gelagert. Um verschieden große Vorratsflaschen aufnehmen zu können, unterscheiden sich die Flaschenaufnahmen der äußeren und der inneren Arme in ihrem Durchmesser. Zur Aufnahme einer größeren Vorratsflasche wird der Flaschenhals der kopfüber eingeführten großen Vorratsflasche in den Halteabschnitt der äußeren Arme eingesteckt. Die inneren Arme sind dabei aus den äußeren Armen heraus geschwenkt. Zur Aufnahme einer kleineren Vorratsflasche werden die inneren Arme in die äußeren Arme eingeschwenkt, wobei in der Flaschenaufnahme der inneren Arme der Flaschenhals der kopfüber eingeführten kleinen Vorratsflasche eingesteckt wird. Die zwei unterschiedlichen Durchmesser der Flaschenaufnahmen der äußeren und inneren Haltearme ermöglichen die Aufnahme von zwei unterschiedlich großen Vorratsflaschen.

Die nicht veränderlichen Durchmesser der Flaschenaufnahmen der äußeren und inneren Arme lassen nur die Verwendung von zwei Vorratsflaschen bestimmter Größe zu. Es ist daher nicht möglich, kleinere oder größere Vorratsflaschen mit hiervon abweichenden Durchmessern einzusetzen.
Ein weiterer Nachteil der bekannten Flaschenhalter ergibt sich bei der Benutzung der äußeren und inneren Arme. So können beim Einstecken einer großen Vorratsflasche in die Flaschenaufnahme der äußeren Arme unbeabsichtigt die inneren Arme in die äußeren Arme einschwenken, da diese nicht zuverlässig in der ausgeschwenkten Stellung gehalten werden und so das Einstecken der großen Vorratsflasche zusätzlich erschwert.
Darüber hinaus ist der Flaschenhals der Vorratsflasche nicht in der Flaschenaufnahme fixiert, sondern nur in der Flaschenaufnahme eingesteckt.
Aus der US 2011/0266409 A1 ist ein Flaschenhalter für Vorratsflaschen bekannt, der einen Grundkörper mit einer Flaschenaufnahme für eine Vorratsflasche umfasst, wobei in dem Grundkörper zwei Verschiebeschlitze angeordnet sind, die sich um die durch eine Öffnung im Grundkörper gebildete Flaschenaufnahme erstrecken. In den Verschiebeschlitzen werden zwei Klemmbacken geführt, welche jeweils mit einem Hebelarm verbunden sind. Die Hebelarme sind verschwenkbar am Grundkörper angelenkt und entgegen der Rückstellkraft eines Federelements gegeneinander verschwenkbar. Durch Verschwenken der Hebelarme werden die Klemmbacken in den Verschiebeschlitzen linear verschoben.
Eine weitere Flaschenhalterung ist aus der ES 2 397 476 bekannt.
Aufgabe der Erfindung ist es daher, einen Flaschenhalter für eine Injektions- oder Infusionsvorrichtung zu schaffen, mit dem eine möglichst einfache und schnelle Bestückung der Injektionsvorrichtung mit Vorratsflaschen unterschiedlicher Größe ermöglicht werden kann und die Vorratsflaschen sicher im Flaschenhalter gehalten werden.
Diese Aufgabe wird durch eine Vorrichtung mit den Merkmalen des Anspruchs 1 gelöst. Bevorzugte Ausführungsformen dieser Vorrichtung sind in den abhängigen Ansprüchen 2 bis 13 angegeben.

Der erfindungsgemäße Flaschenhalter für eine Injektions- oder Infusionsvorrichtung umfasst einen Grundkörper, welcher wenigstens eine Flaschenaufnahme zur Aufnahme eines Flaschenhalses einer Vorratsflasche aufweist, wobei in dem Grundkörper ein Schacht ausgebildet ist, in dem ein mit einer Feder vorgespannter Spannschieber linear verschiebbar gelagert ist, welcher in einer ersten Stellung den Flaschenhals der Vorratsflasche arretiert und in einer zweiten Stellung den Flaschenhals freigibt. Durch die Verwendung eines Spannschiebers können Vorratsflaschen unterschiedlicher Größe in der Flaschenaufnahme sicher und schnell aufgenommen und arretiert werden.
In einer besonders vorteilhaften Ausgestaltung weist der Spannschieber wenigstens einen Vorsprung auf, der in der ersten Stellung in die Flaschenaufnahme ragt und in der zweiten Stellung außer Eingriff mit der Flaschenaufnahme steht, bzw. nicht in diese ragt. Der Flaschenhalter weist einen Rastmechanismus auf, welchen den Spannschieber in der ersten Position fixiert. Der Rastmechanismus umfasst zweckmäßig eine federnde Nase am Spannschieber und eine Anschlagfläche an einer Wand des Schachts. Der Rastmechanismus fixiert den Spannschieber in der ersten Stellung im Schacht, indem die federnde Nase an der Anschlagfläche einrastet und dadurch die lineare Verschiebung des durch die Feder vorgespannten Spannschiebers begrenzt. Die Anordnung der Nase und der dazu korrespondierenden Anschlagfläche kann auch umgekehrt ausgebildet sein.
Der Spannschieber ist zweckmäßig durch einen äußeren Druck auf den Spannschieber gegen die Rückstellkraft der Feder in die zweite Stellung bringbar. Der äußere Druck kann von einem Bediener der Vorrichtung manuell aufgebracht werden. In einer zweckmäßigen Ausführungsform ist an dem Grundkörper ein Griff vorgesehen, welcher beim manuellen Einschieben des Spannschiebers in die Aussparung als Gegendrucklager dienen kann.
In bevorzugter Ausgestaltung weist der Grundkörper einen ersten und einen zweiten Arm auf, welche starr ausgebildet sind. Alternativ können die Arme jedoch auch gegeneinander leicht federnd ausgebildet sein. Beim Einschieben bzw. Einstecken des Flaschenhalses der Vorratsflasche werden die beiden Arme auseinander gedrückt und halten auf Grund der Federwirkung den Flaschenhals zusätzlich zum Spannschieber. Somit können Herstellungsungenauigkeiten bei der Vorratsflasche wie etwa ein breiterer Flaschenhals ausgeglichen werden.

In einer weiteren vorteilhaften Ausführung weist der Flaschenhalter eine zweite Flaschenaufnahme auf, welche zweckmäßig wie die erste Flaschenaufnahme als Ausnehmung, insbesondere als Bohrung, im Grundkörper ausgebildet ist. Die zweite Flaschenaufnahme ermöglicht die Aufnahme weiterer unterschiedlich großer Vorratsflaschen, insbesondere kleinerer Vorratsflaschen.

Diese und weitere Vorteile des erfindungsgemäßen Flaschenhalters für eine Injektions- oder Infusionsvorrichtung ergeben sich aus dem nachfolgend unter Bezugnahme auf die begleitende Zeichnung näher beschriebenen Ausführungsbeispiel. Es zeigen:
- **Figur 1**: eine Schnittdarstellung einer Explosionsansicht eines erfindungsgemäßen Flaschenhalters;
- **Figur 2a**: eine Schnittdarstellung des erfindungsgemäßen Flaschenhalters von Figur 1 in einer ersten Stellung;
- **Figur 2b**: eine Schnittdarstellung des erfindungsgemäßen Flaschenhalters von Figur 1 in einer zweiten Stellung;
- **Figur 3**: eine perspektivische Darstellung der Explosionsansicht eines erfindungsgemäßen Flaschenhalters mit Gegendrucklager;
- **Figur 4a**: eine perspektivische Darstellung eines erfindungsgemäßen Flaschenhalters von Figur 3 mit einer 20 ml Vorratsflasche;
- **Figur 4b**: eine perspektivische Darstellung des erfindungsgemäßen Flaschenhalters von Figur 3 mit einer 100 ml Vorratsflasche;
- **Figur 4c**: eine perspektivische Darstellung des erfindungsgemäßen Flaschenhalters von Figur 3 mit einer 500 ml Vorratsflasche und
- **Figur 4d**: eine perspektivische Darstellung des erfindungsgemäßen Flaschenhalters von Figur 3 mit einer 1000 ml Vorratsflasche.

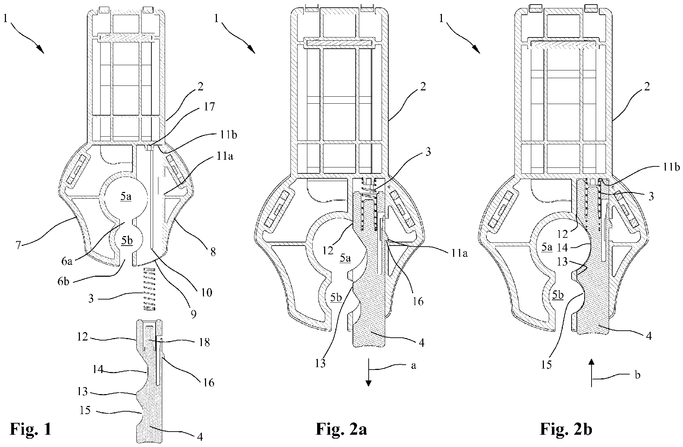

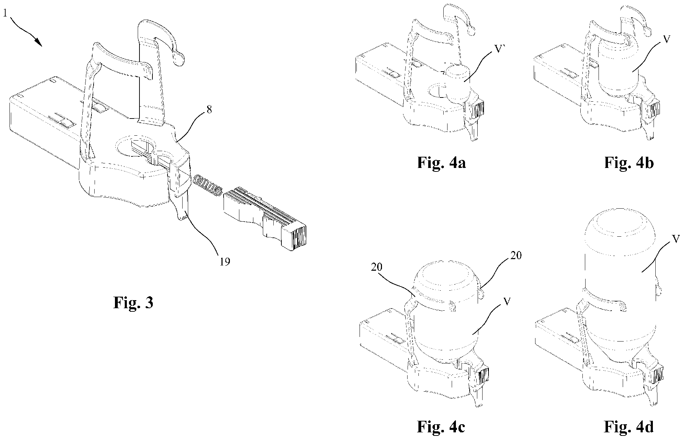

In Figur 1 ist eine Explosionsansicht eines erfindungsgemäßen Flaschenhalters 1 für eine Injektions- oder Infusionsvorrichtung mit einem Grundkörper 2, einer Feder 3 und einem Spannschieber 4 dargestellt. Zur Aufnahme eines Flaschenhalses einer (in Figur 4 gezeigten) Vorratsflasche V weist der Grundkörper 2 eine erste Flaschenaufnahme 5a und eine zweite Flaschenaufnahme 5b auf, welche als Ausnehmung, insbesondere als Bohrung im Grundkörper 2 ausgebildet sind. Der Durchmesser der ersten Flaschenaufnahme 5a ist größer als der Durchmesser der zweiten Flaschenaufnahme 5b. In die erste und zweite Flaschenaufnahme 5a, 5b kann jeweils der Flaschenhals einer Vorratsflasche V kopfüber eingesteckt werden.

Zwischen den Ausnehmungen der Flaschenaufnahmen 5a, 5b erstreckt sich ein Kanal 6a. Zudem ist ein weiterer Kanal 6b vorgesehen, der sich von der Flaschenaufnahme 5b durch den Grundkörper 2 nach außen erstreckt. Durch die Kanäle 6a, 6b und die Flaschenaufnahmen 5a, 5b weist der Grundkörper 2 eine zangenähnliche Ausgestaltung in Form eines ersten Arms 7 und eines zweiten Arms 8 auf. Die Arme 7, 8 sind leicht elastisch federnd gegeneinander ausgebildet. Die Flaschenaufnahmen 5a, 5b und die Kanäle 6a, 6b sind dabei in einer Reihe angeordnet, wie aus den Figuren 1 und 2 ersichtlich ist.

Aufgrund der Kanäle 6a, 6b kann eine bereits mit einem (nicht gezeigten) Zuführschlauch verbundene Vorratsflasche V seitlich in den Flaschenhalter 1 eingeführt und in die Flaschenaufnahme 5a bzw. 5b eingesteckt werden. Die Kanäle 6a, 6b dienen also zur Durchführung des Zuführschlauchs.

Im Bereich des zweiten Arms 8 ist ein linear verlaufender Schacht 9 in Form einer Aussparung im Grundkörper 2 ausgebildet. Der Schacht 9 schneidet hierbei die Randbereiche der Flaschenaufnahmen 5a, 5b und ist zu diesen hin zumindest teilweise geöffnet. Ferner ist im Schacht 9 an einer äußeren Wand, insbesondere im Boden, ein Führungssteg 10 angeordnet, welcher sich annähernd über die komplette Länge des Schachts 9 erstreckt. Im Schacht 9 ist darüber hinaus eine seitliche erste Anschlagfläche 11a und eine stirnseitige zweite Anschlagfläche 11b vorgesehen.

Der Spannschieber 4 weist an seiner Unterseite eine (nicht gezeigte) zum Führungssteg 10 korrespondierende Nut auf. Seitlich ist am Spannschieber 4 ein erster Vorsprung 12 und einer zweiter Vorsprung 13 ausgebildet, wobei sich an den Vorsprüngen 12, 13 eine erste Einbuchtung 14 und eine zweite Einbuchtung 15 anschließen. An einer den Vorsprüngen 12, 13 gegenüberliegenden Seite des Spannschiebers 4 ist eine federnde Nase 16 ausgebildet. Die Anschlagsfläche 11a und die federnde Nase 16 bilden zusammen einen Rastmechanismus, welche in den nachfolgenden Figuren genauer erläutert wird.

Zur Montage des Flaschenhalters 1 wird zunächst die Feder 3 in den Schacht 9 eingeschoben oder eingesetzt, wobei an der stirnseitigen Schachtwand ein Aufnahmezapfen 17 zur Aufnahme der Feder 3 angeformt ist. Anschließend wird der Spannschieber 4 in den Schacht 9 geschoben, sodass der Führungssteg 10 in der Nut des Spannschiebers 4 verläuft und diesen sicher in dem Schacht 9 führt. Auch am Spannschieber 4 ist eine Federaufnahme in Form eines Zapfens 18 vorgesehen, über den ein Endbereich der Feder 3 geführt wird.

In den Schnittdarstellungen von Figur 2 ist der wie zuvor beschrieben montierte Flaschenhalter mit der in den Grundkörper 2 des Flaschenhalters 1 eingesetzten Feder 3 und dem Spannschieber 4 in einer ersten Stellung (Figur 2a) und in einer zweiten Stellung (Figur 2b) gezeigt.

In der in Figur 2a gezeigten ersten Stellung wird der in den Schacht 9 eingeschobene Spannschieber 4 aufgrund der Feder 3 aus dem Schacht 9 in Pfeilrichtung a herausgedrückt bzw. verschoben. Zur Fixierung des Spannschiebers 4 ist der Rastmechanismus vorgesehen, der den Spannschieber 4 in der ersten Stellung fixiert, indem die federnde Nase 16 des Spannschiebers 4 an der Anschlagfläche 11a einrastet und so die lineare Verschiebung des durch die Feder 3 vorgespannten Spannschiebers 4 begrenzt. In der ersten Stellung des Spannschiebers 4 ragt der erste Vorsprung 12 in die Flaschenaufnahme 5a und der zweite Vorsprung 13 ragt in die Flaschenaufnahme 5b. Ein in die Flaschenaufnahme 5a bzw. 5b eingesteckter Flaschenhals einer Vorratsflasche V wird durch die Klemmwirkung des in die jeweilige Flaschenaufnahme 5a bzw. 5b eingreifenden Vorsprungs 12 bzw. 13 des Spannschiebers 4 somit in der Flaschenaufnahme 5a bzw. 5b arretiert.

Wie in Figur 2b angedeutet, ist der Spannschieber 4 durch einen äußeren Druck auf den Spannschieber 4 in Pfeilrichtung b gegen die Rückstellkraft der Feder 3 in die zweite Stellung bringbar. Die lineare Verschiebung des Spannschiebers 4 in den Schacht 9 gegen die Rückstellkraft der Feder 3 ist dabei durch die stirnseitige zweite Anschlagfläche 11b des Schachts 9 begrenzt. Die Form der Einbuchtung 14 am Spannschieber 4 ist dabei der Form der ersten Flaschenaufnahme 5a und die Form der Einbuchtung 15 der Form der zweiten Flaschenaufnahme 5b angepasst. Bei der gezeigten kreisförmigen Ausnehmung ist die Form der Einbuchtung kreissegmentförmig. Auf Grund dieser Ausgestaltung der Einbuchtungen 14, 15 des Spannschiebers 4 kann in der zweiten Stellung des Spannschiebers 4 der Flaschenhals der Vorratsflasche V in eine der Flaschenaufnahmen 5a, 5b eingesetzt werden bzw. ein in der Flaschenaufnahme 5a, 5b arretierter Flaschenhals der Vorratsflasche V freigegeben werden. Die zur Arretierung des Flaschenhalses der Vorratsflasche V vorgesehen Vorsprünge 12 und 13 sind im Schacht 9 in der zweiten Stellung somit außer Eingriff mit einer der Flaschenaufnahmen 5a, 5b.

Wie aus der Beschreibung der ersten und zweiten Stellung des Spannschiebers 4 hervorgeht, wird der Spannschieber 4 durch die Feder 3 immer in die erste Stellung, also die Verriegelungsstellung, gedrückt. Die federnde Rastnase 16 dient hierbei als Anschlag, damit der Spannschieber 4 nicht aus dem Schacht 9 herausfällt bzw. herausgedrückt wird. In der zweiten Stellung, also der Freigabestellung, hält der Spannschieber 4 lediglich durch Festhalten des Bedieners oder durch Klemmung an einem Flaschenhals der Vorratsflasche 4.

Zur einhändigen Bedienung des Flaschenhalters 1 ist ein in Figur 3 gezeigter Griff 19 vorgesehen, welcher an dem zweiten Arm 8 des Grundkörpers 2 angeordnet ist. Der Griff 19 dient als Gegendrucklager beim manuellen Einschieben des Spannschiebers 4 in den Schacht 9.

In den Darstellungen der Figur 4 ist die Verwendung unterschiedlich großer Vorratsflaschen V, V' ersichtlich. So können in der zweiten Flaschenaufnahme 5b kleine Vorratsflaschen V' mit einem Füllvermögen von beispielsweise 20 ml aufgenommen werden (Figur 4a), während in der ersten Flaschenaufnahme 5a Vorratsflaschen V mit einem Füllvermögen von beispielsweise 100 ml (Figur 4b), 500 ml (Figur 4c) und 1000 ml (Figur 4d) eingesetzt werden können.

Um die in der ersten Flaschenaufnahme 5a aufgenommene Vorratsflasche V zusätzlich zu sichern, sind, wie aus den Figuren 4c und 4d ersichtlich, am Grundkörper 2 bzw. dem ersten und zweiten Arm 7, 8 des Flaschenhalters 1 flexible Halteelemente 20 angeordnet, welche die in der Flaschenaufnahme 5a aufgenommene Vorratsflasche V zumindest teilweise kraftschlüssig und/oder formschlüssig an ihrem Außenumfang umgreifen.

Die Erfindung ist nicht auf das beschriebene Ausführungsbeispiel beschränkt. So kann der erfindungsgemäße Flaschenhalter nicht nur in einer Injektionsvorrichtung zur Injektion von Kontrastmitteln sondern beispielsweise auch in Infusionsgeräten verwendet werden. Anders als bei dem beschriebenen Ausführungsbeispiel können statt einer ersten und zweiten Flaschenaufnahme 5a, 5b je nach Anwendungsfall auch nur eine Flaschenaufnahme oder auch noch weitere Flaschenaufnahmen im Grundkörper vorgesehen sein. Es ist auch möglich, einen erfindungsgemäßen Flaschenhalter zur Aufnahme einer Vorratsflasche zusammen mit einem herkömmlichen Beutelhalter zur Aufnahme eines eine zu injizierende Flüssigkeit enthaltenden Beutel zu verwenden.

Der Flaschenhalter 1 ist zudem nicht auf die Aufnahme der Vorratsflaschen mit den zuvor beschriebenen Füllvermögen begrenzt.

## Patentansprüche

1. Flaschenhalter (1) für eine Injektions- oder Infusionsvorrichtung mit einem Grundkörper (2), welcher wenigstens eine Flaschenaufnahme (5a) zur Aufnahme eines Flaschenhalses einer Vorratsflasche aufweist, wobei in dem Grundkörper (2) ein Schacht (9) ausgebildet ist, in dem ein mit einer Feder (3) vorgespannter Spannschieber (4) linear verschiebbar gelagert ist, welcher in einer ersten Stellung den Flaschenhals der Vorratsflasche arretiert und in einer zweiten Stellung den Flaschenhals freigibt, **gekennzeichnet durch** einen Rastmechanismus, welcher den Spannschieber (4) in der ersten Position fixiert.

2. Flaschenhalter nach Anspruch 1, **dadurch gekennzeichnet, dass** der Spannschieber (4) wenigstens einen Vorsprung (12, 13) aufweist, der in der ersten Stellung in die Flaschenaufnahme (5a) ragt und in der zweiten Stellung außer Eingriff mit der Flaschenaufnahme (5a) steht.

3. Flaschenhalter nach Anspruch 1 oder 2, **dadurch gekennzeichnet, dass** der Rastmechanismus eine federnde Nase (16) am Spannschieber (4) und eine Anschlagfläche (11a) an einer Wand des Schachts (9) umfasst.

4. Flaschenhalter nach Anspruch 3, **dadurch gekennzeichnet, dass** der Rastmechanismus den Spannschieber (4) in der ersten Stellung im Schacht (9) fixiert, indem die federnde Nase (16) an der Anschlagfläche (11a) einrastet und dadurch die lineare Verschiebung des durch die Feder (3) vorgespannten Spannschiebers (4) begrenzt.

5. Flaschenhalter nach einem der voranstehenden Ansprüche, **dadurch gekennzeichnet, dass** der Spannschieber (4) durch einen äußeren Druck auf den Spannschieber (4) gegen die Rückstellkraft der Feder (3) in die zweite Stellung bringbar ist.

6. Flaschenhalter nach einem der voranstehenden Ansprüche, **dadurch gekennzeichnet, dass** der Spannschieber (4) wenigstens eine Einbuchtung (14, 15) aufweist, welche in ihrer Form der Form der Flaschenaufnahme (5a) angepasst ist.

7. Flaschenhalter nach einem der voranstenden Ansprüche, **dadurch gekennzeichnet, dass** der Grundkörper (2) einen ersten und zweiten Arm (7; 8) aufweist.

8. Flaschenhalter nach einem der voranstehenden Ansprüche, **dadurch gekennzeichnet, dass** an dem Grundkörper (2) einen Griff (19) vorgesehen ist, welcher beim Einschieben des Spannschiebers (4) in den Schacht (9) als Gegendrucklager dient.

9. Flaschenhalter nach einem der voranstehenden Ansprüche, **dadurch gekennzeichnet, dass** der Flaschenhalter (1) eine zweite Flaschenaufnahme (5b) aufweist.

10. Flaschenhalter nach Anspruch 9, **dadurch gekennzeichnet, dass** der Spannschieber (4) eine zweite Einbuchtung (15) aufweist, welche in ihrer Form der Form der zweiten Flaschenaufnahme (5b) angepasst ist.

11. Flaschenhalter nach einem der voranstehenden Ansprüche, **dadurch gekennzeichnet, dass** der Schacht (9) als Aussparung im Grundkörper (2) ausgebildet ist.

12. Flaschenhalter nach einem der voranstehenden Ansprüche, **dadurch gekennzeichnet, dass** die wenigstens eine Flaschenaufnahme (5a, 5b) als Ausnehmung, insbesondere als Bohrung, im Grundkörper (2) ausgebildet ist.

13. Flaschenhalter nach einem der voranstehenden Ansprüche, **dadurch gekennzeichnet, dass** im Schacht (9) ein Führungssteg (10) ausgebildet ist, welcher in den Spannschieber (4) eingreift.

## Claims

1. Bottle holder (1) for an injection or infusion device with a base unit (2) which has at least one bottle receiving opening (5a) for receiving a bottle neck of a supply bottle, wherein the base unit (2) has a shaft (9) formed therein in which a clamping slide (4) pre-loaded with a spring (3) is mounted so as to be displaceable linearly and in a first position locks the bottle neck of the supply bottle and in a second position releases the bottle neck, **characterised by** a latching mechanism which immobilises the clamping slide (4) in the first position.

2. Bottle holder according to claim 1, **characterised in that** the clamping slide (4) has at least one projection (12, 13) which in the first position projects into the bottle receiving opening (5a) and in the second position is disengaged from the bottle receiving opening (5a).

3. Bottle holder according to claim 1 or 2, **characterised in that** the latching mechanism comprises a springy catch (16) on the clamping slide (4) and a stop face (11a) on a wall of the shaft (9).

4. Bottle holder according to claim 3, **characterised in that** the latching mechanism immobilises the clamping slide (4) in the first position in the shaft (9) **in that** the springy catch (16) engages at the stop face (11a) and thereby limits the linear displacement of the clamping slide (4) pre-loaded by the spring (3).

5. Bottle holder according to one of the preceding claims, **characterised in that** the clamping slide (4) can be brought into the second position by an external pressure on the clamping slide (4) against the restoring force of the spring (3).

6. Bottle holder according to one of the preceding claims, **characterised in that** the clamping slide (4) has at least one recess (14, 15) which in its shape is matched to the shape of the bottle receiving opening (5a).

7. Bottle holder according to one of the preceding claims, **characterised in that** the base unit (2) has a first arm and a second arm (7; 8).

8. Bottle holder according to one of the preceding claims, **characterised in that** a handle (19) is provided on the base unit (2), which handle serves as a support when pushing the clamping slide (4) into the shaft (9).

9. Bottle holder according to one of the preceding claims, **characterised in that** the bottle holder (1) has a second bottle receiving opening (5b).

10. Bottle holder according to claim 9, **characterised in that** the clamping slide (4) has a second recess (15) which in its shape is matched to the shape of the second bottle receiving opening (5b).

11. Bottle holder according to one of the preceding claims, **characterised in that** the shaft (9) is embodied in the form of an opening in the base unit (2).

12. Bottle holder according to one of the preceding claims, **characterised in that** the at least one bottle receiving opening (5a, 5b) is embodied in the form of a recess, in particular a bore, in the base unit (2).

13. Bottle holder according to one of the preceding claims, **characterised in that** a guide (10) is formed in the shaft (9), which guide engages in the clamping slide (4).

## Revendications

1. Support de bouteilles (1) pour un dispositif d'injection ou d'infusion avec un corps de base (2), lequel.présente au moins un logement de bouteille (5a) pour la réception d'un col de bouteille d'une bouteille de réserve, dans lequel dans le corps de base (2), un puits (9) est réalisé, dans lequel un coulisseau de serrage (4) précontraint avec un ressort (3) est logé de manière linéairement déplaçable, lequel bloque le col de bouteille de la bouteille de réserve dans une première position et libère le col de bouteille dans une deuxième position, **caractérisé par** un mécanisme d'encliquetage, qui fixe le coulisseau de serrage (4) dans la première position.

2. Support de bouteilles selon la revendication 1, **caractérisé en ce que** le coulisseau de serrage (4) présente au moins une saillie (12, 13), qui dépasse dans le logement de bouteille (5a) dans la première position et est hors de prise avec le logement de bouteille (5a) dans la deuxième position.

3. Support de bouteilles selon la revendication 1 ou 2, **caractérisé en ce que** le mécanisme d'encliquetage comprend un nez élastique (16) au niveau du coulisseau de serrage (4) et une surface de butée (11a) au niveau d'une paroi du puits (9).

4. Support de bouteilles selon la revendication 3, **caractérisé en ce que** le mécanisme d'encliquetage fixe le coulisseau de serrage (4) dans la première position dans le puits (9), par le fait que le nez élastique (16) s'encliquète au niveau de la surface de butée (11a) et limite ainsi le déplacement linéaire du coulisseau de serrage (4) précontraint par le ressort (3).

5. Support de bouteilles selon l'une quelconque des revendications précédentes, **caractérisé en ce que** le coulisseau de serrage (4) peut être amené dans la deuxième position par une pression extérieure sur le coulisseau de serrage (4) contre la force de rappel du ressort (3).

6. Support de bouteilles selon l'une quelconque des revendications précédentes, **caractérisé en ce que** le coulisseau de serrage (4) présente au moins une partie concave (14, 15), dont la forme est adaptée à la forme du logement de bouteille (5a)

7. Support de bouteilles selon l'une quelconque des revendications précédentes, **caractérisé en ce que** le corps de base (2) présente un premier et deuxième bras (7 ; 8).

8. Support de bouteilles selon l'une quelconque des revendications précédentes, **caractérisé en ce qu'**une poignée (19) est prévue au niveau du corps de base (2), laquelle sert de palier de contre-pression lors de l'insertion du coulisseau de serrage (4) dans le puits (9).

9. Support de bouteilles selon l'une quelconque des revendications précédentes, **caractérisé en ce que** le support de bouteilles (1) présente un deuxième logement de bouteille (5b).

10. Support de bouteilles selon la revendication 9, **caractérisé en ce que** le coulisseau de serrage (4) présente une deuxième partie concave (15), dont la forme est adaptée à la forme du deuxième logement de bouteille (5b).

11. Support de bouteilles selon l'une quelconque des revendications précédentes, **caractérisé en ce que** le puits (9) est réalisé sous forme de cavité dans le corps de base (2).

12. Support de bouteilles selon l'une quelconque des revendications précédentes, **caractérisé en ce que** l'au moins un logement de bouteille (5a, 5b) est réalisé sous forme d'évidement, en particulier d'alésage, dans le corps de base (2).

13. Support de bouteilles selon l'une quelconque des revendications précédentes, **caractérisé en ce qu'**une nervure de guidage (10) est réalisée dans le puits (9), laquelle vient en prise dans le coulisseau de serrage (4).
